# EUROPÄISCHE PATENTANMELDUNG

(11) **EP 1 325 758 A2**
(43) Veröffentlichungstag der Anmeldung: **09.07.2003**
(21) Anmeldenummer: 02024749.0
(22) Anmeldetag: 06.11.2002
(51) Int. Cl.: A61L 31/16

(54) **Beschichtungssystem**

(30) Priorität: 08.01.2002 DE 10200388
(71) Anmelder: Translumina GmbH, 72379 Hechingen (DE)
(72) Erfinder: Schratzenstaller, Thomas, 85375 Neufahrn (DE); Wintermantel, Erich, Prof. Dr. med. Dr.-Ing. habil, 85402 Kranzberg (DE)
(74) Vertreter: Laufhütte, Dieter, Dr.-Ing.

(57) **Zusammenfassung**

Die vorliegende Erfindung betrifft ein Beschichtungssystem zum Aufbringen eines oder mehrerer Medikamente auf einen Stent. Eine besonders genaue Dosierung des Medikamentes auf dem Stent wird erfindungsgemäß dadurch erzielt, daß wenigstens eine Sprüheinrichtung sowie eine Rotationseinheit vorgesehen ist, die mit der Sprüheinrichtung oder dem zu beschichtenden Stent mittelbar oder unmittelbar in Verbindung steht oder verbindbar ist und durch die der Stent relativ zur Sprüheinrichtung rotierbar ist.

Die vorliegende Erfindung betrifft ferner ein Beschichtungssystem zum Aufbringen eines oder mehrerer Medikamente auf einen Stent, das durch wenigstens eine Sprüheinrichtung sowie Mittel zur Erzeugung einer Potentialdifferenz zwischen der Sprüheinrichtung und dem Stent gekennzeichnet ist.

Die vorliegende Erfindung betrifft ferner ein Verfahren zum Aufbringen eines oder mehrerer Medikamente sowie ein Verfahren zur individualisierten Medikamentendosierung auf einen Stent.

Die vorliegende Erfindung betrifft schließlich einen Schrumpfschlauch zum Aufbringen von Medikamenten auf einen Stent sowie ein Verfahren zum Aufbringen eines Stents auf einen Katheter und/oder zum Aufbringen von Medikamenten auf einen Stent.

## Beschreibung

Die vorliegende Erfindung betrifft ein Beschichtungssystem zum Aufbringen eines oder mehrerer Medikamente auf einen Stent.

Die Beschichtung von Stents mit Medikamenten hat sich in der Vergangenheit als günstig erwiesen, um Restenosen durch Proliferation des Gewebes weitgehend zu verhindern oder zumindest wesentlich zu verzögern. Beispielhaft werden als Medikamente Heparin oder auch Rapamycin genannt, die auf den Außen- und/oder Innenumfang der Stents aufgebracht werden. Zur Aufnahme der Medikamente können ferner kanalförmige Vertiefungen in den Verstrebungen des Stents vorgesehen sein, wie dies beispielsweise aus der EP 0 950 386 A2 bekannt ist. Bei der Beschichtung von Stents mit Medikamenten ist eine hohe Präzision erforderlich, da die Dosierung sowie die Verteilung des Medikamentes auf dem Stent für den Behandlungserfolg von erheblicher Bedeutung sind. Ein weiterer wesentlicher Parameter ist die Applikationsform der Medikamente. Diese können beispielsweise mit biokompatiblen Polymeren beschichtet werden, wodurch einerseits die Fixierung der Medikamente auf dem Stent verbessert wird und andererseits auf die Kinetik der Freisetzung des Medikamentes Einfluß genommen werden kann. Von Bedeutung ist somit nicht nur ein hohe Dosiergenauigkeit hinsichtlich des Medikaments, sondern auch hinsichtlich des Polymers. Dies gilt sowohl für Fälle in denen das Polymer zur Beschichtung des Medikaments verwendet wird, als auch für die Verwendung des Polymers als Polymermatrix, in der das Medikament gleichmäßig verteilt ist.

Es ist die Aufgabe der vorliegenden Erfindung ein Beschichtungssystem der eingangs genannten Art bereitzustellen, mit dem die Beschichtung eines Stents mit hoher Präzision möglich ist.

Diese Aufgabe wird durch ein Beschichtungssystem mit den Merkmalen des Patentanspruchs 1 gelöst. Danach ist wenigstens eine Sprüheinrichtung sowie eine Rotationseinheit vorgesehen, die mit der Sprüheinrichtung oder dem zu beschichtenden Stent mittelbar oder unmittelbar in Verbindung steht oder verbindbar ist und durch die der Stent relativ zur Sprüheinrichtung rotierbar ist. Entscheidend ist eine Relativbewegung zwischen Stent und Sprüheinrichtung, wobei der Stent, die Sprüheinrichtung oder beides rotierbar angeordnet sein können. Denkbar wäre beispielsweise eine motorisch rotierbare Platte, auf der der Stent angeordnet ist sowie eine ortsfeste Sprüheinrichtung, die während der Rotation des Stents betätigt wird. Hierdurch wird eine gleichmäßige Verteilung des Medikamentes bzw. des Polymers auf dem Stent ermöglicht. Die Sprüheinrichtung kann beispielsweise mit einer einfachen Schnappverbindung (snap-in-Verbindung) in einer Halterung montiert werden, was den Vorteil aufweist, daß diese leicht gegen eine Sprüheinrichtung austauschbar ist, mit der ein weiteres Medikament oder eine Polymerbeschichtung aufgetragen werden soll.

Die Sprüheinrichtung kann derart ausgeführt sein, daß das Medikament in Pulveroder Tröpfchenform auf dem Stent aufgebracht wird. Üblich ist es, das Medikament in einem geeigneten Lösungsmittel, wie z. B. Wasser, Alkohol oder Aceton zu lösen und anschließend der Sprüheinrichtung zuzuführen.

Gemäß einer bevorzugten Ausgestaltung der vorliegenden Erfindung ist vorgesehen, daß die Düse der Sprüheinrichtung derart ausgeführt ist, daß eine Beschichtung des Stents über dessen gesamte Länge erfolgt. In diesem Falle ist es nicht erforderlich, daß der Stent in einer Richtung parallel zur Rotationsachse der Rotationseinheit bewegt wird, da auch ohne eine derartige Bewegung eine gleichmäßige Beschichtung möglich ist.

In weiterer Ausgestaltung der vorliegenden Erfindung ist vorgesehen, daß der Stent auf einem Katheter aufgebracht ist. Zum Einsatz des Stents ist ohnehin die Anordnung auf einem Ballonkatheter erforderlich. Gemäß dieser bevorzugten Ausgestaltung der vorliegenden Erfindung wird der Stent nicht nach der Beschichtung, sondern davor auf einen Katheter aufgeschrumpft und in diesem Zustand in das Beschichtungssystem eingeführt. Dabei kann das Katheterröhrchen aufgewickelt und derart abgedeckt sein, daß eine Beschichtung des Katheterröhrchens unterbleibt. In diesem Fall ist sichergestellt, daß nur der Stent und eventuell die freiliegenden Bereiche des Ballonkatheters beschichtet werden.

Grundsätzlich kann ebenso vorgesehen sein, den Stent im nicht gekrimpten Zustand zu beschichten. Allgemein kann die Beschichtung des Stents somit vor dem Krimpen oder auch, wie oben ausgeführt, nach dessen Aufschrumpfen auf den Katheter erfolgen.

Ferner kann vorgesehen sein, daß die Rotationseinheit eine rohrförmige rotierende Aufnahme umfaßt, in der das Katheterröhrchen fixiert ist. Das Katheterröhrchen kann beispielsweise in die Aufnahme eingeklebt werden. In einer derartigen Ausführung ist eine ausreichende Fixierung des Stents gewährleistet, ohne daß der Stent selbst auf der Rotationseinheit fixiert werden müßte.

Vorzugsweise ist eine Vakuumpumpe vorgesehen, mittels derer wenigstens im Bereich des Stents sowie der Sprüheinrichtung ein Unterdruck erzeugbar ist.

Besonders vorteilhaft ist es, wenn mehrere Sprüheinrichtungen vorgesehen sind, die in Drehrichtung der Rotationseinheit voneinander beabstandet angeordnet sind. In diesem Falle ist ein modularer Aufbau mit mehreren Sprüheinrichtungen denkbar, wobei vorgesehen sein kann, daß die unterschiedlichen Sprüheinrichtungen mit unterschiedlichen Medikamenten bzw. mit einem oder verschiedenen Polymeren befüllt sind. Beispielsweise ist denkbar, daß der Stent zunächst mit einem ersten Medikament, anschließend mit einer aus Polymer bestehenden Sperrschicht, anschließend mit einem weiteren Medikament und schließlich wieder mit einer Sperrschicht beschichtet wird. Bei dieser Ausgestaltung der vorliegenden Erfindung ist es nicht erforderlich, daß die Sprüheinrichtungen entsprechend der Abfolge der Schichten ausgewechselt werden, da die unterschiedlichen Sprüheinrichtungen mit unterschiedlichen Medikamenten bzw. Polymeren betrieben werden. Alternativ oder zusätzlich kann vorgesehen sein, daß mehrere Sprüheinrichtungen vorgesehen sind, die in Richtung der Drehachse der Rotationseinheit voneinander beabstandet angeordnet sind. Auf diese Weise ist es möglich, in Längsrichtung unterschiedliche Beschichtungen aufzubringen oder auch verhältnismäßig lange Stents zu beschichten, ohne daß eine Verschiebung der Sprüheinrichtung oder des Stents erforderlich ist.

Jedoch können ferner Mittel vorgesehen sein, durch die der Stent in Richtung der Drehachse der Rotationseinheit bewegbar ist.

Gemäß einer bevorzugten Ausgestaltung der vorliegenden Erfindung ist eine Steuereinheit vorgesehen, mittels der die zeitliche Abfolge der Aktivierung der Sprüheinrichtungen und/oder die Bewegung des Stents relativ zu den Sprüheinrichtungen steuerbar ist. Auf diese Weise ist ein Beschichtungsautomat realisierbar, in dem die Art, die Dosierung sowie die Applikationsform des oder der Medikamente eingegeben wird und der im Wesentlichen vollautomatisch eine Beschichtung des Stents gemäß diesen Vorgaben durchführt. Die Steuereinheit kann gemäß den Vorgaben des Benutzers den Beschichtungsautomaten beispielsweise derart steuern, daß der Beschichtungserfolg hinsichtlich der gewünschten Dicke der Polymerbeschichtung, der Art und Dosierung der Medikamente, des bevorzugten Orts der Beschichtung etc. erzielt wird.

Die vorliegende Erfindung betrifft ferner ein Beschichtungssystem zum Aufbringen eines oder mehrerer Medikamente auf einen Stent, das durch wenigstens eine Sprüheinrichtung sowie Mittel zur Erzeugung einer Potentialdifferenz zwischen der Sprüheinrichtung und dem Stent gekennzeichnet ist. Auf diese Weise läßt sich eine besonders homogene und gleichmäßige Verteilung des Medikamentes bzw. Polymers auf dem Stent realisieren. Hierzu ist es nicht zwingend erforderlich, daß eine Rotationsbewegung des Stents relativ zur Sprüheinrichtung erfolgt. Diese kann jedoch zusätzlich vorgesehen sein.

Besonders bevorzugt ist es, wenn der Stent geerdet ist und gegenüber der oder den Sprüheinrichtungen ein positives Potential aufweist. Die Potentialdifferenz hat die Erzeugung von Feldlinien zur Folge, entlang derer sich die die Sprüheinrichtung verlassenden Partikel aus Medikament und/oder Polymer bewegen. Die Feldlinien umgeben den Stent vollständig, so daß auch eine Beschichtung des Stents auf der von der Sprüheinrichtung abgewandten "Rückseite" erfolgt. Dadurch wird der Vorteil erreicht, daß eine Rotation, wie oben angeführt, nicht zwingend erforderlich ist. Die Sprüheinrichtung kann beispielsweise bei bis zu 100 kV betrieben werden.

Durch das Anliegen einer Potentialdifferenz zwischen Stent und Sprüheinrichtung läßt sich ein gleichmäßiges Verteilen des Medikamentes sowie die Erzielung besonders dünner Schichten realisieren.

In bevorzugter Ausgestaltung ist das Beschichtungssystem gemäß einem der Ansprüche 1 bis 11 ausgeführt. Insbesondere könne auch mehrere Sprüheinrichtungen vorgesehen sein, die in Rotationsrichtung und/oder in Richtung der Drehachse voneinander beabstandet sind. Zusätzlich zum Aufbringen eines Feldes kann vorgesehen sein, daß eine Rotationsbewegung des Stents relativ zu der oder den Sprüheinrichtungen vorgenommen wird. Die Beschichtung des Stents kann im nicht gekrimpten Zustand oder auch beispielsweise nach dem Aufschrumpfen des Stents auf einen Katheter vorgenommen werden.

Die vorliegende Erfindung betrifft ferner ein Verfahren zum Aufbringen eines oder mehrerer Medikamente auf einen Stent, das durch die Schritte Einbringen des Stents in eine Vorrichtung gemäß einem der Ansprüche 1 bis 14, Rotation der Rotationseinheit und/oder Erzeugung einer Potentialdifferenz zwischen Stent und der oder den Sprüheinrichtungen und Aufbringen des Medikamentes durch Betätigung der Sprüheinrichung gekennzeichnet ist.

Die vorliegende Erfindung betrifft ferner ein Verfahren zur individualisierten Medikamentendosierung auf einen Stent, das durch die Schritte Bestimmung der für einen Patienten erforderlichen Art, Applikationsform und/oder Dosis des auf dem Stent aufzubringenden Medikaments und Betätigung einer oder mehrerer das aufzubringende Medikament aufweisenden Auftragseinrichtung derart, daß die Sollwerte hinsichtlich Art, Applikationsform und/oder Dosierung des Medikaments auf dem Stent erzielt werden gekennzeichnet ist. Ein derartiges Verfahren hat den wesentlichen Vorteil, daß eine sehr genaue Dosierung des Medikaments in Abhängigkeit von dem tatsächlichen Bedarf möglich ist. Das Verfahren kann vorteilhafterweise unmittelbar vor dem Einsetzen des Stents angewendet werden. Während herkömmliche Stents mit einer fertigen Beschichtung und somit mit im Wesentlichen vorgegebenen Dosierungswerten angeboten werden, bietet das vorliegende Verfahren die Möglichkeit, die Dosierung, Art, Applikation etc. des Medikamentes individuell und damit abgestimmt auf die tatsächlichen Patientenbedürfnisse einzustellen. Die Zeitdauer zwischen Aufbringen des Medikaments auf den Stent und dem Einsetzen desselben wird erheblich verkürzt. Darüber hinaus wird das Zulassungsverfahren, das nur isoliert für das Medikament und den Stent zu durchlaufen ist, vereinfacht.

Die Auftragseinrichtung umfaßt vorzugsweise ein Beschichtungssystem nach einem der Ansprüche 1 bis 14. Denkbar sind Beschichtungsautomaten oder auch das manuelle Aufbringen der Beschichtung vor dem Einsetzen des Stents. Im Falle des Einsatzes von Beschichtungsautomaten kann vorgesehen sein, daß der Benutzer Art, Dosis und Applikationsform des oder der Medikamente eingibt und der Beschichtungsvorgang nach Eingabe dieser Parameter vollautomatisch abläuft. Auch kann vorgesehen sein, daß patientenbezogene Parameter, wie z. B. Alter, Gewicht und krankheitsbezogene Parameter eingegeben werden und der Beschichtungsautomat anhand dieser Daten eine für den individuellen Fall ideale Beschichtung wählt. Voraussetzung dafür sind selbstverständlich verläßliche Daten, aus denen sich für diese Beschichtung erforderlichen Korrelationen zwischen patienten- bzw. krankheitsbezogenen Daten und der Art und Zusammensetzung der Beschichtung ergeben. Derartige Daten und Modelle sind beispielsweise in einer Speichereinheit der Steuerung des Beschichtungsautomaten abgelegt.

Auch ist es möglich, den Ort der Dosierung individuell zu wählen. Infrage kommt eine Beschichtung auf der Außen- und/oder Innenseite des Stents, in den Endbereichen oder in einem zentralen Bereich oder gleichmäßig über die gesamte Länge. Für eine Beschichtung auf der Innenseite kann vorgesehen sein, daß eine Auftragseinrichtung, insbesondere eine Sprühvorrichtung derart angeordnet ist, daß ihre Düse mit der Längsachse des Stents fluchtet.

Beispielsweise kann das Aufbringen des Medikaments während des Anliegens einer Potentialdifferenz zwischen Auftragseinrichtung und dem Stent erfolgen. Der Stent wird vor dem Aufbringen des Medikamentes erwärmt und geerdet und nach dem Aufbringen des Medikamentes getrocknet und steht dann für den Einsatz zur Verfügung. Selbstverständlich kann alternativ oder zusätzlich vorgesehen sein, daß das Aufbringen des Medikamentes während einer Rotationsbewegung des Stents erfolgt.

Auf dem Stent kann ein Polymer aufgebracht werden, das die oben genannten Funktionen einer Sperrschicht erfüllt. Es kann eine Mischung aus Polymer und Medikament oder es kann das Polymer getrennt von dem Medikament aufgebracht werden.

Darüber hinaus kann vorgesehen sein, daß die Beschichtung auf der Außen- und Innenseite des Stents erfolgt oder vorzugsweise in Vertiefungen, die in den Verstrebungen der Wandung des Stents aufgenommen sind.

Die vorliegende Erfindung betrifft ferner einen Schrumpfschlauch zum Aufbringen von Medikamenten auf einen Stent. Der Schrumpfschlauch ist dadurch gekennzeichnet, daß er bei der Erwärmung eine Verringerung seines Innendurchmessers erfährt. Erfindungsgemäß befinden sich im Schrumpfschlauch oder auf der Innenseite des Schrumpfschlauches oder auf einem auf der Innenseite des Schrumpfschlauches befindlichen Träger Medikamente. Auf diese Weise wird es möglich, einen Stent auf einen Katheter bzw. davon unabhängig oder gleichzeitig Medikamente auf einen Stent aufzubringen. Im Gegensatz zu vorbekannten Verfahren, bei denen eine im Wesentlichen linienförmige mechanische Krafteinleitung erfolgt, wird gemäß der vorliegenden Erfindung die Homogenität der Krafteinleitung gewährleistet. In Umfangsrichtung des Stents kommt es somit zu einer gleichmäßigen Krafteinleitung, so daß der Stent homogen und fest auf dem Katheter aufgebracht wird bzw. die Medikamente gleichförmig an den gewünschten Bereichen auf den Stent aufgebracht werden. Ein Absplittern von Substanz durch ungleichmäßige Verformung des Stents aufgrund lokal unterschiedlicher Belastungen kann somit vermieden werden.

Der Träger kann als Trägerfolie ausgeführt sein. In dem Fall der Verwendung eines Medikamententrägers kann die Innenseite des Schrumpfschlauches nicht strukturiert ausgeführt sein.

Ferner kann vorgesehen sein, daß der Schrumpfschlauch auf seiner Innenseite eine strukturierte Oberfläche aufweist, in deren Vertiefungen oder Ausnehmungen die Medikamente aufgenommen sind. Dabei können die Medikamente mechanisch in den Vertiefungen oder Ausnehmungen fixiert sein. Diese mechanische Verbindung wird gelöst, wenn der Schrumpfschlauch durch Erwärmen auf den Stent aufgepreßt wird. Die Medikamente werden dabei aus dem Schrumpfschlauch freigegeben und auf den Stent bzw. in Vertiefungen auf der Stentoberfläche eingebracht.

In weiterer Ausgestaltung der vorliegenden Erfindung sind Klebemitteldepots vorgesehen, die gemeinsam mit den Medikamenten auf den Stent auftragbar sind und mittels derer die Medikamente auf dem Stent fixiert werden. Beim Aufschrumpfen des Schlauches wird aus den Klebemitteldepots das Klebemittel freigesetzt und bewirkt, daß die Medikamente auf dem Stent an der gewünschten Position fixiert werden.

Der Schrumpfschlauch oder der auf dessen Innenseite befindliche Träger kann Aussparungen aufweisen, die sich mit den Aussparungen des Stents decken. Stents üblicher Bauart weisen eine Vielzahl von miteinander verbundenen Verstrebungen auf, zwischen denen Aussparungen gebildet werden. Die Verstrebungen dienen dazu, ein Aufweiten sowie ein Aufschrumpfen des Stents zu ermöglichen. Es kann nun vorgesehen sein, daß auch der Schrumpfschlauch bzw. der Träger entsprechend strukturiert ist, d. h. daß sich die Aussparungen im Schrumpfschlauch bzw. dem Träger mit den Aussparungen des Stents decken.

In weiterer Ausgestaltung der vorliegenden Erfindung sind im Schrumpfschlauch oder auf dessen Innenseite ein oder mehrere, vorzugsweise zwei, drei oder vier, Reißmittel, insbesondere Reißdrähte oder Reißfäden, mittels derer der Schrumpfschlauch nach dem Schrumpfvorgang auftrennbar ist. Die vorzugsweise auf der Innenseite des Schlauches vorgesehenen Reißmittel dienen dazu, den Schrumpfschlauch nach dem Aufschrumpfen wieder zu entfernen. Hierzu werden die Reißmittel, vorzugsweise Reißdrähte, betätigt, wodurch der Schrumpfschlauch aufgerissen wird und anschließend abgenommen werden kann. Es kann sich um einen Reißdraht, Reißfaden etc. handeln. Vorzugsweise sind jedoch mehrere derartiger Reißmittel vorgesehen.

Gemäß diesem Aspekt der vorliegenden Erfindung wird eine Vereinfachung des Aufbringens von Medikamenten bzw. eine Vereinfachung des Aufbringens des Stents auf den Katheter erzielt. Insbesondere läßt sich eine Homogenität der Krafteinleitung erreichen, was sich insbesondere hinsichtlich der Gleichmäßigkeit und Zuverlässigkeit des Aufbringens der Medikamente positiv auswirkt. Wesentliche Parameter, die auf das Schrumpfen des Schlauches Einfluß haben, sind die Dicke des Schrumpflauches, der Vernetzungsgrad des den Schrumpfschlauch bildenden Polymers sowie die geometrische Ausführung des Stents. Bei der Wahl der geeigneten Dicke der Schrumpfschlauchwandung ist zu berücksichtigen, daß es ein Dickenoptimum gibt. Während zu dünne Schläuche ungeeignet sind, da die erforderlichen Kräfte nicht aufgebracht werden können, sind zu dicke Schläuche verhältnismäßig starr und lassen nur geringe Verformungen zu. Dies kann dazu führen, daß die Freigabe der Medikamente oder das Aufbringen des Trägers nicht oder nur unvollständig erfolgt.

Der vorzugsweise aus Polymer bestehende Schrumpfschlauch besitzt vorzugsweise auf seiner Innenseite Medikamente oder einen Medikamente enthaltenden Träger. Hierbei handelt es sich beispielsweise um eine Trägerfolie (Drug Printing Release Device), die nach dem Aufbringen auf dem Stent verbleibt. Die Trägerfolie besteht vorzugsweise aus einem biodegradablen Material, kann aber auch nicht degradabel ausgeführt sein.

Die vorliegende Erfindung betrifft ferner ein Verfahren zum Aufbringen eines Stents auf einen Katheter und/oder zum Aufbringen von Medikamenten auf einen Stent. Gemäß diesem Verfahren wird ein Schrumpfschlauch über den Stent geschoben und der Schrumpfschlauch anschließend erwärmt. Hierdurch kommt es zu dem oben genannten Vorteil einer homogenen Krafteinleitung, so daß der Stent fest auf dem Katheter anliegt bzw. die Medikamente fest und in der gewünschten Dosierung auf den Stent aufgebracht werden. Bei dem verwendeten Schrumpfschlauch handelt es sich vorzugsweise um einen Schrumpfschlauch nach einem der Ansprüche 22 bis 28.

In weiterer Ausgestaltung der vorliegenden Erfindung ist vorgesehen, daß im Schrumpfschlauch oder auf dessen Innenseite, ein oder mehrere Reißmittel, insbesondere Reißdrähte oder Reißfäden, vorgesehen sind, und daß der Schrumpfschlauch nach dem Schrumpfvorgang mittels der Reißmittel aufgetrennt wird.

Besonders vorteilhaft ist es, wenn sich im Schrumpfschlauch oder auf der Innenseite des Schrumpfschlauches Medikamente befinden, die beim Erwärmen des Schrumpfschlauches auf den Stent aufgebracht werden.

In weiterer Ausgestaltung der vorliegenden Erfindung ist vorgesehen, daß die Medikamente auf dem Stent durch Klebemittel fixiert werden, die beim Schrumpfen des Schlauches mit den Medikamenten aus dem Schrumpfschlauch austreten.

Besonders vorteilhaft ist es, wenn ein Medikamente enthaltender Träger, insbesondere eine Trägerfolie vorgesehen ist, die auf der Innenseite des Schrumpfschlauches angeordnet ist und die beim Erwärmen des Schrumpfschlauches auf den Stent aufgepreßt wird.

Nach dem erfindungsgemäßen Verfahren kann der Stent auf den Katheter aufgebracht werden und es kann zudem erreicht werden, daß die Medikamente in der gewünschten Dosierung und Applikationsform auf den Stent aufgetragen werden.

Darüber hinaus ist im Gegensatz zu vorbekannten Verfahren eine Automatisierung möglich. Denkbar wäre beispielsweise, nicht nur einen Stent, sondern eine Vielzahl von Stents gleichzeitig mit einem durchgehenden Schrumpfschlauch zu überziehen, anschließend den Schrumpfvorgang einzuleiten und danach mittels der Reißmittel den Schrumpfschlauch wieder abzutrennen. Auf diese Weise ist es möglich, gleichzeitig eine Vielzahl von Stents bereitzustellen.

Weitere Einzelheiten und Vorteile der vorliegenden Erfindung werden in einem in der Zeichnung dargestellten Ausführungsbeispiel näher erläutert:

Es zeigen:
- Fig. 1:: eine schematische Darstellung einer Sprüheinrichtung sowie eines Stents mit zwischen Sprüheinrichtung und Stent angelegter Potentialdifferenz und
- Fig. 2:: einen schematischen Verfahrensablauf zur Herstellung einsatzfertiger Stents.

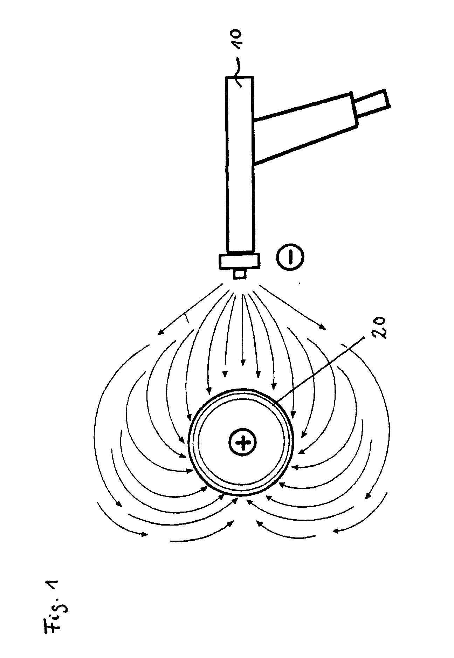

Fig. 1 zeigt in einer schematischen Darstellung die als Spritzpistole ausgeführte Sprüheinrichtung 10, die bei bis zu 100 kV betrieben wird. An der Sprüheinrichtung 10 liegt ein negatives Potential an.

Ferner dargestellt ist der Stent 20, der geerdet ist und somit gegenüber dem Potential der Sprüheinrichtung 10 ein positives Potential aufweist.

Der Stent 20 ist im Wesentlichen zylindrisch ausgeführt und in seiner Draufsicht bzw. Querschnittsansicht in Fig. 1 dargestellt. Der Stent 20 weist eine Verstrebungen umfassende Wandung auf, in denen Vertiefungen zur Aufnahme des oder der Medikamente vorgesehen sein können. Außer der Sprüheinrichtung 10 können weitere Sprüheinrichtungen vorgesehen sein, die in Umfangsrichtung oder parallel zur Längsachse des Stents 10 voneinander beabstandet sind.

Aufgrund der Potentialdifferenz zwischen Sprüheinrichtung 10 und Stent 20 bilden sich Feldlinien aus, entlang derer sich der Beschichtungsstoff, d. h. das in einem Lösungsmittel gelöste Medikament nach dem Verlassen der Sprüheinrichtung bewegt, wie dies durch die Pfeile in Fig. 1 dargestellt ist. Daraus ergibt sich der Vorteil, daß eine Rotation des Stents 20 nicht erforderlich ist, um auch die Rückseite des Stents 20 zu beschichten, da sich die Feldlinien im Wesentlichen gleichmäßig auf den Umfang des Stents 20 verteilen. Die Beschichtungsvorrichtung wird daher vereinfacht. Die Vorrichtung gemäß Fig. 1 ermöglicht eine gleichmäßige Verteilung des Medikamentes und darüber hinaus die Aufbringung dünner Schichten.

Mit der in Fig. 1 dargestellten Vorrichtung kann nicht nur das in einem Lösungsmittel vorliegende Medikament, sondern auch ein Polymer aufgetragen werden. Dies kann gemeinsam mit dem Medikament, beispielsweise in Form einer Polymermatrix, oder auch davon getrennt aufgetragen werden.

Zusätzlich kann vorgesehen sein, den Stent 20 in eine Rotationsbewegung zu versetzten, wobei die Rotationsachse senkrecht zur Papierebene steht und sich damit parallel zur Längsachse des Stents 20 erstreckt.

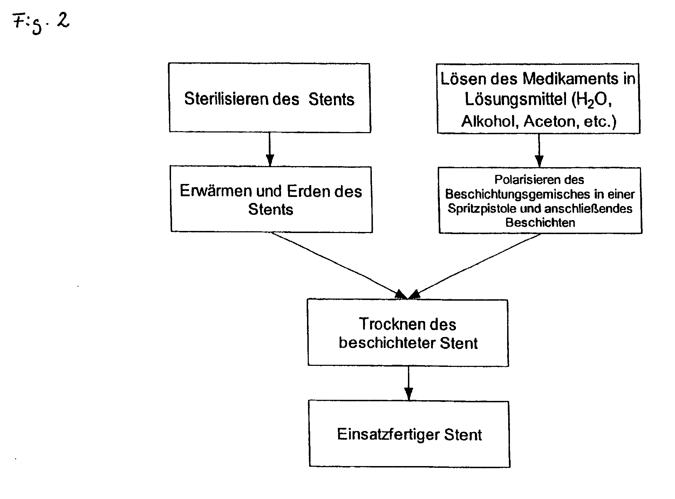

Fig. 2 zeigt einen Verfahrensablauf zur Bereitstellung eines einsatzfertigen Stents. Zunächst wird der Stent sterilisiert und in das Beschichtungssystem eingebracht. Dort wird der Stent erwärmt und geerdet.

Parallel dazu erfolgt das Lösen des Medikamentes in einem geeigneten Lösungsmittel, wie beispielsweise Wasser, Alkohol oder Aceton. Vor Beginn des Beschichtungsvorganges wird die Sprüheinrichtung 10 polarisiert. Im Anschluß an den Beschichtungsvorgang wird der beschichtete Stent getrocknet und steht dann zum Einsatz zur Verfügung. Dabei kann vorgesehen sein, daß der Stent bereits in seinem auf einen Ballonkatheter aufgeschrumpften Zustand der Beschichtung unterzogen wird. In diesem Falle bedarf es keiner weiteren Verfahrensschritte, um den Stent nun zum Einsatz zur Verfügung zu stellen.

## Patentansprüche

1. Beschichtungssystem zum Aufbringen eines oder mehrerer Medikamente auf einen Stent,
**gekennzeichnet durch**
wenigstens eine Sprüheinrichtung sowie eine Rotationseinheit, die mit der Sprüheinrichtung oder dem zu beschichtenden Stent mittelbar oder unmittelbar in Verbindung steht oder verbindbar ist und **durch** die der Stent relativ zur Sprüheinrichtung rotierbar ist.

2. Beschichtungssystem nach Anspruch 1, **dadurch gekennzeichnet, daß** die Sprüheinrichtung derart ausgeführt ist, daß das Medikament in Pulver- oder Tröpfchenform auf den Stent aufgebracht wird.

3. Beschichtungssystem nach Anspruch 1 oder 2, **dadurch gekennzeichnet, daß** die Düse der Sprüheinrichtung derart ausgeführt ist, daß eine Beschichtung des Stents über dessen gesamte Länge erfolgt.

4. Beschichtungssystem nach einem der Ansprüche 1 bis 3, **dadurch gekennzeichnet, daß** der Stent auf einem Katheter aufgebracht ist.

5. Beschichtungssystem nach Anspruch 4, **dadurch gekennzeichnet, daß** das Katheterröhrchen aufgewickelt und derart abgedeckt ist, daß eine Beschichtung des Katheterröhrchens unterbleibt.

6. Beschichtungssystem nach Anspruch 4, **dadurch gekennzeichnet, daß** die Rotationseinheit eine rohrförmige rotierende Aufnahme umfaßt, in der das Katheterröhrchen fixiert ist.

7. Beschichtungssystem nach einem der Ansprüche 1 bis 6, **dadurch gekennzeichnet, daß** eine Vakuumpumpe vorgesehen ist, mittels derer wenigstens im Bereich des Stents sowie der Sprüheinrichtung ein Unterdruck erzeugbar ist.

8. Beschichtungssystem nach einem der Ansprüche 1 bis 7, **dadurch gekennzeichnet, daß** mehrere Sprüheinrichtungen vorgesehen sind, die in Drehrichtung der Rotationseinheit voneinander beabstandet angeordnet sind.

9. Beschichtungssystem nach einem der Ansprüche 1 bis 8, **dadurch gekennzeichnet, daß** mehrere Sprüheinrichtungen vorgesehen sind, die in Richtung der Drehachse der Rotationseinheit voneinander beabstandet sind.

10. Beschichtungssystem nach einem der Ansprüche 1 bis 9, **dadurch gekennzeichnet, daß** Mittel vorgesehen sind, durch die der Stent in Richtung der Drehachse der Rotationseinheit bewegbar ist.

11. Beschichtungssystem nach einem der Ansprüche 1 bis 10, **dadurch gekennzeichnet, daß** eine Steuereinheit vorgesehen ist, mittels der die zeitliche Abfolge der Aktivierung der Sprüheinrichtungen und/oder die Bewegung des Stents relativ zu den Sprüheinrichtungen steuerbar ist.

12. Beschichtungssystem zum Aufbringen eines oder mehrerer Medikamente auf einen Stent, **gekennzeichnet durch** wenigstens eine Sprüheinrichtung sowie Mittel zur Erzeugung einer Potentialdifferenz zwischen der Sprüheinrichtung und dem Stent.

13. Beschichtungssystem nach Anspruch 12, **dadurch gekennzeichnet, daß** der Stent geerdet ist und gegenüber der Sprüheinrichtung ein positives potential aufweist.

14. Beschichtungssystem nach Anspruch 12 oder 13, **dadurch gekennzeichnet, daß** das Beschichtungssystem gemäß einem der Ansprüche 1 bis 11 ausgeführt ist.

15. Verfahren zum Aufbringen eines oder mehrerer Medikamente auf einen Stent, **gekennzeichnet durch** die Schritte: Einbringen des Stents in eine Vorrichtung gemäß einem der Ansprüche 1 bis 14, Rotation der Rotationseinheit und/oder Erzeugung einer Potentialdifferenz zwischen Stent und der oder den Sprüheinrichtungen und Aufbringen des Medikamentes **durch** Betätigung der Sprüheinrichtung.

16. Verfahren zur individualisierten Medikamentendosierung auf einen Stent, **gekennzeichnet durch** die Schritte: Bestimmung der für einen Patienten erforderlichen Art, Applikationsform und/oder Dosis des auf dem Stent aufzubringenden Medikaments und Betätigung einer oder mehrerer das aufzubringende Medikament aufweisenden Auftragseinrichtungen derart, daß die Sollwerte hinsichtlich Art, Applikationsform und/oder Dosierung des Medikamentes auf dem Stent erzielt werden.

17. Verfahren nach Anspruch 16, **dadurch gekennzeichnet, daß** die Auftragseinrichtung ein Beschichtungssystem nach einem der Ansprüche 1 bis 14 umfaßt.

18. Verfahren nach einem der Ansprüche 15 bis 17, **dadurch gekennzeichnet, daß** das Aufbringen des Medikamentes während des Anliegens einer Potentialdifferenz zwischen Auftragseinrichtung und dem Stent erfolgt und daß der Stent vor dem Aufbringen des Medikamentes erwärmt und geerdet wird und nach dem Aufbringen des Medikamentes getrocknet wird.

19. Verfahren nach einem der Ansprüche 16 bis 18, **dadurch gekennzeichnet, daß** das Aufbringen des Medikamentes während einer Rotationsbewegung des Stents erfolgt.

20. Verfahren nach einem der Ansprüche 15 bis 19, **dadurch gekennzeichnet, daß** auf dem Stent ein Polymer aufgebracht wird.

21. Verfahren nach Anspruch 20, **dadurch gekennzeichnet, daß** eine Mischung aus Polymer und Medikament oder daß das Polymer getrennt von dem Medikament aufgebracht wird.

22. Schrumpfschlauch zum Aufbringen von Medikamenten auf einen Stent, **dadurch gekennzeichnet, daß** sich im Schrumpfschlauch oder auf der Innenseite des Schrumpfschlauches oder auf einem auf der Innenseite des Schrumpfschlauches befindlichen Träger Medikamente befinden.

23. Schrumpfschlauch nach Anspruch 22, **dadurch gekennzeichnet, daß** der Träger als Trägerfolie ausgeführt ist.

24. Schrumpfschlauch nach Anspruch 22 oder 23, **dadurch gekennzeichnet, daß** der Schrumpfschlauch auf seiner Innenseite eine strukturierte Oberfläche aufweist, in deren Vertiefungen oder Ausnehmungen die Medikamente aufgenommen sind.

25. Schrumpfschlauch nach Anspruch 24, **dadurch gekennzeichnet, daß** die Medikamente mechanisch in den Vertiefungen oder Ausnehmungen fixiert sind.

26. Schrumpfschlauch nach einem der Ansprüche 22 bis 25, **dadurch gekennzeichnet, daß** Klebemitteldepots vorgesehen sind, die gemeinsam mit den Medikamenten auf den Stent auftragbar sind und mittels derer die Medikamente auf dem Stent fixiert werden.

27. Schrumpfschlauch nach einem der Ansprüche 22 bis 26, **dadurch gekennzeichnet, daß** der Schrumpfschlauch oder der auf dessen Innenseite befindliche Träger Aussparungen aufweist, die sich mit den Aussparungen des Stents decken.

28. Schrumpfschlauch nach einem der Ansprüche 22 bis 27, **dadurch gekennzeichnet, daß** im Schrumpfschlauch oder auf dessen Innenseite ein oder mehrere Reißmittel, insbesondere Reißdrähte oder Reißfäden, vorgesehen sind, mittels derer der Schrumpfschlauch nach dem Schrumpfvorgang auftrennbar ist.

29. Verfahren zum Aufbringen eines Stents auf einen Katheder und/oder zum Aufbringen von Medikamenten auf einen Stent, **dadurch gekennzeichnet, daß** ein Schrumpfschlauch über den Stent geschoben wird und der Schrumpfschlauch anschließend erwärmt wird.

30. Verfahren nach Anspruch 29, **dadurch gekennzeichnet, daß** im Schrumpfschlauch oder auf dessen Innenseite ein oder mehrere Reißmittel, insbesondere Reißdrähte oder Reißfäden, vorgesehen sind, und daß der Schrumpfschlauch nach dem Schrumpfvorgang mittels der Reißmittel aufgetrennt wird.

31. Verfahren nach Anspruch 29 oder 30, **dadurch gekennzeichnet, daß** sich im Schrumpfschlauch oder auf der Innenseite des Schrumpfschlauches Medikamente befinden, die beim Erwärmen des Schrumpfschlauches auf den Stent aufgebracht werden.

32. Verfahren nach Anspruch 31, **dadurch gekennzeichnet, daß** die Medikamente auf dem Stent durch Klebemittel fixiert werden, die beim Schrumpfen des Schlauches mit den Medikamenten aus dem Schrumpfschlauch austreten.

33. Verfahren nach Anspruch 29 oder 30, **dadurch gekennzeichnet, daß** ein Medikamente enthaltender Träger, insbesondere eine Trägerfolie vorgesehen ist, die auf der Innenseite des Schrumpfschlauches angeordnet ist und die beim Erwärmen des Schrumpfschlauches auf den Stent aufgepreßt wird.
